(19) Europäisches Patentamt / European Patent Office / Office européen des brevets

(11) **EP 4 667 517 A1**

(12) **EUROPEAN PATENT APPLICATION**
published in accordance with Art. 153(4) EPC

(43) Date of publication:
24.12.2025 Bulletin 2025/52

(21) Application number: 24788586.6

(22) Date of filing: 28.03.2024

(51) International Patent Classification (IPC):
$C08J\ 11/16^{(2006.01)}$  $A61F\ 13/15^{(2006.01)}$
$A61F\ 13/53^{(2006.01)}$  $A61L\ 15/24^{(2006.01)}$
$A61L\ 15/60^{(2006.01)}$

(52) Cooperative Patent Classification (CPC):
A61F 13/15; A61F 13/53; A61L 15/24; A61L 15/60;
C08J 11/16

(86) International application number:
PCT/JP2024/012891

(87) International publication number:
WO 2024/214558 (17.10.2024 Gazette 2024/42)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 13.04.2023 JP 2023065575

(71) Applicant: Sumitomo Seika Chemicals Co., Ltd.
Kako-gun, Hyogo 675-0145 (JP)

(72) Inventors:
• HINAYAMA, Tetsuhiro
  Himeji-shi, Hyogo 672-8076 (JP)
• HAYASHI, Yoshiki
  Himeji-shi, Hyogo 672-8076 (JP)

(74) Representative: Vossius & Partner
Patentanwälte Rechtsanwälte mbB
Siebertstrasse 3
81675 München (DE)

(54) **WATER-ABSORBENT RESIN PARTICLES AND PRODUCTION METHOD THEREFOR, ABSORBENT BODY, AND ABSORBENT ARTICLE**

(57) A production method for water-absorbent resin particles containing a crosslinked polymer includes a preparation step of preparing a polymer solution that contains a polymer and a solvent, and a crosslinking step of crosslinking the polymer through a covalent bond to form the crosslinked polymer. The water-absorbent resin particles have a dissolution component index of $2 \times 10^{-3}$ to $6 \times 10^{-3}$, which is represented by the following equation.

$$\text{dissolution component index} = \text{dissolved matter (g/g)/CRC (g/g)}$$

**Fig.1**

EP 4 667 517 A1

## Description

## Technical Field

The present invention relates to water-absorbent resin particles and a method for producing the same, an absorbent body, and an absorbent article.

## Background Art

Generally, disposable sanitary products are configured to contain pulp fibers and a water-absorbent resin between a water-impermeable cover sheet and a water-permeable non-woven fabric, and the water-absorbent resin swells by containing moisture so that excrement can be absorbed. After such sanitary products are used, the sanitary products are treated by incineration or landfilling, but in recent years, the collection and reuse of components from used sanitary products have been examined. For example, Patent Literature 1 discloses a technique for decomposing a water-absorbent resin with a focus on cutting of a crosslinked portion in the water-absorbent resin, and a technique for collecting a water-soluble recycled polymer obtained by decomposing a water-absorbent resin as an aggregate by crosslinking with a multivalent metal ion.

## Citation List

## Patent Literature

[Patent Literature 1] Japanese Unexamined Patent Publication No. 2020-49398

## Summary of Invention

## Problem to be Solved

In many techniques disclosed as methods of regenerating a water-absorbent resin collected from used sanitary products, the water-absorbing ability of the water-absorbent resin is compared before and after a predetermined treatment, and in a case where the water-absorbing ability is more than or equal to the water-absorbing ability before the treatment, it is considered that the water-absorbent resin has been regenerated. However, since a crosslinked structure of the water-absorbent resin is destroyed by the predetermined treatment, the polymer component is likely to dissolve out, it is necessary to maintain the water-absorbing ability and to suppress an increase in the amount of dissolved polymer. However, in Patent Literature 1, it cannot be said that both the maintenance of the water-absorbing ability and the suppression of the dissolution of the polymer are achieved.

One aspect of the present invention relates to a method for producing water-absorbent resin particles having high water absorption performance and a small amount of dissolved matter, which is applicable to regenerating water-absorbent resin particles from a polymer formed by chemically decomposing a water-absorbent resin.

## Solution to Problem

One aspect of the present disclosure includes, for example, the following means.

[1] A production method for water-absorbent resin particles containing a crosslinked polymer, the production method including: a preparation step of preparing a polymer solution that contains a polymer and a solvent; and a crosslinking step of crosslinking the polymer through a covalent bond to form the crosslinked polymer.

[2] The production method according to [1], in which the preparation step includes cleaving a crosslinked structure of a crosslinked polymer in a water-absorbent resin for recycling that contains the crosslinked polymer, to obtain the polymer.

[3] The production method according to [2], in which the preparation step further includes collecting the water-absorbent resin for recycling from an absorbent article.

[4] The production method according to any one of [1] to [3], in which the polymer has a weight-average molecular weight of 100,000 or more.

[5] The production method according to any one of [1] to [4], in which in the crosslinking step, the polymer solution is gelled.

[6] The production method according to any one of [1] to [5], in which the polymer contains a carboxyl group, and the polymer is crosslinked by a reaction between the carboxyl group and a crosslinking agent and/or a reaction between the carboxyl groups.

[7] The production method according to any one of [1] to [6], in which the polymer is crosslinked through at least one group selected from the group consisting of a carboxylic acid ester group, a thioester group, an amide group, an acid anhydride group, an oxyalkylene group, and an oxyarylene group.

[8] Water-absorbent resin particles, in which a dissolution component index represented by the following equation is 2 $\times 10^{-3}$ to 6 $\times 10^{-3}$.

dissolution component index = dissolved matter (g/g)/CRC (g/g)

[9] An absorbent body including: the water-absorbent resin particles according to [8].

[10] An absorbent article including: the absorbent body according to [9].

Advantageous Effects of **Invention**

According to one aspect of the present invention, it is possible to provide a method for producing water-absorbent resin particles having high water absorption performance and a small amount of dissolved matter, which is applicable to the regeneration of water-absorbent resin particles from a polymer formed by chemically decomposing a water-absorbent resin.

**Brief Description of Drawings**

Fig. 1 is a cross-sectional view showing an example of an absorbent article having an absorbent body.

Fig. 2 is a schematic view showing a non-woven fabric bag prepared in a case for measuring a centrifuge retention capacity.

**Description of Embodiments**

The present invention is not limited to the following examples.

In the present specification, "(meth)acryl" means both acrylic and methacrylic. The terms "acrylate" and "methacrylate" also similarly denote "(meth)acrylate". The same also applies to other similar terms. The term "(poly)" includes both cases with and without the "poly" prefix. In the numerical value ranges described in a stepwise manner in the present specification, the upper limit or lower limit of a numerical value range in a certain step can be optionally combined with an upper limit or lower limit of a numerical value range in another step. In the numerical value ranges described in the present specification, an upper limit or lower limit of a numerical value range may be replaced with a value shown in examples. The materials described in the present specification may be used alone or in combination with two or more kinds thereof. "Physiological saline " denotes a 0.9 mass% sodium chloride aqueous solution. "Standard sieve" denotes a test sieve (metal mesh sieve) specified in JIS Z 8801-1:2019. Unless otherwise specified, examples and comparative examples are carried out at 1 atm in a normal-temperature and normal-humidity environment, various parameters disclosed in the present specification are values measured in the same environment, and the temperature of various samples is the normal-temperature. "One atmosphere" is 101325 Pa, "normal temperature" is 25°C, and "normal humidity" is 50% RH.

As an example of the production method for water-absorbent resin particles, the method includes a preparation step of preparing a polymer solution containing a polymer and a solvent, and a crosslinking step of crosslinking the polymer in the polymer solution through a covalent bond to form a crosslinked polymer. The water-absorbent resin particles contain a crosslinked polymer. According to the method, it is possible to regenerate water-absorbent resin particles from a polymer formed by chemically decomposing (specifically, cleaving a crosslinked structure of the water-absorbent resin) the water-absorbent resin and to produce water-absorbent resin particles having high water absorption performance and a small amount of dissolved matter. The water-absorbent resin to be subjected to chemical decomposition (hereinafter, also referred to as "water-absorbent resin for recycling") is preferably a used water-absorbent resin (that is, a water-absorbent resin in a gel state due to liquid absorption), but may be an unused water-absorbent resin (for example, a waste of the water-absorbent resin generated in a step of producing the water-absorbent resin), or may be a mixture of both the used and unused water-absorbent resins.

In the preparation step, the polymer in the polymer solution can be obtained, for example, by cleaving the crosslinked structure of the crosslinked polymer of a water-absorbent resin containing a crosslinked polymer. The solvent in the polymer solution may be any solvent as long as the solvent can dissolve the polymer, and examples thereof include water and an organic solvent.

The water-absorbent resin can be, for example, a water-absorbent resin for recycling, which is obtained by crushing an absorbent article. That is, the preparation step may include cleaving a crosslinked structure of a crosslinked polymer of a water-absorbent resin for recycling, which contains a crosslinked polymer, to obtain a polymer or may further include collecting the water-absorbent resin for recycling from an absorbent article.

Examples of the absorbent article include diapers (for example, paper diapers), toilet training pants, incontinence pads, sanitary materials (sanitary napkins, tampons, and the like), sweat pads, pet sheets, portable toilet members, and animal excrement treatment materials. Used absorbent articles may also be employed. The water-absorbent resin for recycling, which is collected from the used water absorbent article, may form a gel by absorbing liquid during use.

The water-absorbent resin for recycling contains, for example, a polymer (crosslinked polymer) of an ethylenically unsaturated monomer. Examples of the ethylenically unsaturated monomer include (meth)acrylic acid and a salt thereof, 2-(meth)acrylamide-2-methylpropanesulfonic acid and a salt thereof, (meth)acrylamide, N,N-dimethyl (meth) acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. The ethylenically unsaturated monomer may include at least one compound selected from the group consisting of acrylic acid and a salt thereof, methacrylic acid and a salt thereof, acrylamide, methacrylamide, and N,N-dimethylacrylamide.

The crosslinked polymer may contain a monomer other than the ethylenically unsaturated monomer. The proportion of the ethylenically unsaturated monomer unit (particularly, (meth)acrylic acid and a salt thereof) in the polymer may be 70 to 100 mol% with respect to the total amount of the monomer units. The proportion of the (meth)acrylic acid and the salt thereof in the ethylenically unsaturated monomer may be 70 to 100 mol%.

The water-absorbent resin for recycling contains, for example, a crosslinked polymer having a poly(meth)acrylic acid structure. Examples of such a crosslinked polymer include a polymer obtained by polymerizing a monomer composition containing (meth)acrylic acid and a crosslinking agent capable of forming a covalent bond by reacting with a carboxyl group of (meth)acrylic acid, a polymer obtained by polymerizing a monomer composition containing (meth) acrylic acid to obtain a polymer and treating a surface of the polymer with a crosslinking agent capable of forming a covalent bond by reacting with a carboxyl group of the polymer, and a polymer obtained by polymerizing a monomer composition containing (meth)acrylic acid and a crosslinking agent capable of forming a covalent bond by reacting with a carboxyl group of (meth)acrylic acid to obtain a polymer and treating a surface of the polymer with a crosslinking agent capable of forming a covalent bond by reacting with a carboxyl group of the polymer.

The shape of the water-absorbent resin for recycling is not particularly limited, and may be an amorphous crushed shape, a scale-like shape, a granular shape, or the like.

The crosslinked structure of the crosslinked polymer of the water-absorbent resin for recycling can be cleaved by bringing the water-absorbent resin for recycling into contact with an acidic component (for example, sulfuric acid), an alkaline component (for example, sodium hydroxide), an oxidizing agent, a reducing agent, or the like. That is, in the preparation step, the crosslinked structure of the crosslinked polymer may be cleaved by bringing the water-absorbent resin for recycling into contact with at least one component selected from the group consisting of an acidic component, an alkaline component, an oxidizing agent, and a reducing agent to obtain a polymer.

The acidic component may be an inorganic acidic component, and may be, for example, at least one inorganic acidic component selected from the group consisting of acetic acid, nitric acid, sulfuric acid, hydrochloric acid, phosphoric acid, and boric acid, or at least one inorganic acidic component selected from sulfuric acid and hydrochloric acid.

The alkaline component may be an inorganic alkaline component, and may be, for example, at least one inorganic alkaline component selected from the group consisting of sodium hydroxide, ammonia, potassium hydroxide, and calcium hydroxide, or at least one inorganic alkaline component selected from the group consisting of sodium hydroxide, potassium hydroxide, and calcium hydroxide.

In a case where the water-absorbent resin for recycling and the acidic component are brought into contact with each other, the temperature of the solution containing the water-absorbent resin for recycling and the acidic component may be 80°C to 140°C. In a case where the water-absorbent resin for recycling and the alkaline component are brought into contact with each other, the temperature of the solution containing the water-absorbent resin for recycling and the alkaline component may be 50°C to 150°C or 50°C to 120°C.

Specifically, for example, in a case where the crosslinked structure of the crosslinked polymer is cleaved by an alkaline component, the crosslinked structure can be cleaved by stirring a solution containing the water-absorbent resin for recycling and the alkaline component at a temperature of 50°C to 150°C and a pH of 10 or greater for 1 hour to 50 hours.

In a case where the water-absorbent resin for recycling contains a crosslinked polymer having a poly(meth) acrylic acid structure, the polymer obtained by cleaving the crosslinked polymer has a poly(meth)acrylic acid structure. In a case where the water-absorbent resin for recycling contains a crosslinked polymer having a poly(meth)acrylic acid structure, from the viewpoint of easily producing water-absorbent resin particles having high water absorption performance and a small amount of dissolved matter by avoiding cutting of the poly(meth)acrylic acid structure, the crosslinked polymer may be cleaved by bringing the water-absorbent resin for recycling into contact with an alkaline component.

The weight-average molecular weight of the polymer obtained in the preparation step may be 100,000 or more, 300,000 or more, 500,000 or more, 700,000 or more, 1,000,000 or more, 1,300,000 or more, 1,500,000 or more, 1,700,000 or more, or 1,900,000 or more. The weight-average molecular weight of the polymer may be 10,000,000 or less, 8,000,000 or less, 6,000,000 or less, 4,000,000 or less, 3,000,000 or less, or 2,000,000 or less. The weight-average molecular weight of the polymer may be 100,000 to 10,000,000, 500,000 to 6,000,000, or 1,000,000 to 3,000,000. The weight-average molecular weight of the polymer can be measured by gel permeation chromatography (GPC) under the following conditions.

· Device: HLC-8320GPC (manufactured by Tosoh Corporation)
·Column: TSKgel Guard Column PWXL, G6000PWXL, GMPWXL, and G3000PWXL (all manufactured by Tosoh Corporation) are connected in this order and measured.
· Detector: differential refractive index detector (RI detector)
· Eluent: 0.2 M $NaNO_3$ aqueous solution
· Flow rate: 1.0 mL/min.
· Column temperature: 60°C
· Sample injection volume: 100 $\mu$L
· Calibration curve: sodium polyacrylate standard (manufactured by Agilent Technologies, Inc.)
· Procedure for preparing sample and measuring sample

A sample solution adjusted to contain 0.01 g of a polymer is placed in a 300 mL beaker, and a 0.2 M NaNO3 aqueous solution as an eluent is added thereto so that the total volume reaches 100 mL, and the solution is stirred at 250 rpm for 1 hour. In a case where the pH of the sample solution is not neutral, the pH is adjusted to 7 using hydrochloric acid (for example, 1 mol/L hydrochloric acid, NAKALAI TESQUE, Inc.) at this time. The sample solution after being stirred is measured by GPC using a filtrate obtained by passing it through a filter syringe having a pore size of 0.8 $\mu$m.

After a polymer is obtained by cleaving the crosslinked structure of the crosslinked polymer, solid-liquid separation may be carried out in order to remove impurities in the polymer solution before carrying out the crosslinking step. For example, the polymer solution may be filtered or ultrafiltered. Before the polymer solution is filtered, the pH of the polymer solution may be adjusted to near neutrality.

From the viewpoint of reducing the viscosity of the polymer solution, dilution, heating, addition of a salt (salt having a monovalent cation), or the like may be performed on the polymer solution after a polymer is obtained by cleaving the crosslinked structure of the crosslinked polymer and before the crosslinking step is performed.

The polymer solution may be subjected to a sterilization treatment after a polymer is obtained by cleaving the crosslinked structure of the crosslinked polymer, and before the crosslinking step is performed.

A polymer dispersion liquid may be obtained by bringing a poor solvent into contact with the polymer solution to precipitate the polymer dissolved in the polymer solution in order to collect the polymer from the polymer solution after a polymer is obtained by cleaving the crosslinked structure of the crosslinked polymer and before the crosslinking step is performed. The purity of the polymer can be increased by performing such an operation. The poor solvent is a solvent in which the solubility of a polymer in 100 g of the solvent at 25°C is less than 0.1 g. The poor solvent may be a water-soluble organic solvent.

Examples of a method of bringing a poor solvent into contact with the polymer solution include a method of adding the poor solvent to the polymer solution and a method of adding the polymer solution to the poor solvent. In a case where a poor solvent is added to the polymer solution, the entire planned amount of the poor solvent to be used may be added to the polymer solution at once, or may be added to the polymer solution over time. Similarly, in a case where a polymer-poor solution is added to a poor solvent, the entire polymer solution may be added to the poor solvent at once, or may be added to the poor solvent over time. The expression "added over time" denotes that a poor solvent (or a polymer solution) is continuously or intermittently added to a polymer solution (or a poor solvent) over a predetermined time. From the viewpoint of suppressing the amount of the poor solvent to be used, a method of adding a poor solvent to the polymer solution over time may be employed.

Examples of the poor solvent include alcohols having 1 to 10 carbon atoms, phenols, ketones, ethers, and nitriles. From the viewpoint of easily obtaining a polymer with higher purity, the poor solvent may be at least one selected from the group consisting of alcohols having 1 to 10 carbon atoms, phenols, and ketones.

Examples of the alcohols having 1 to 10 carbon atoms include methanol, ethanol, propanol, butanol, pentanol, ethylene glycol, and glycerin. Examples of the phenols include phenol, cresol, dibutylhydroxytoluene, and eugenol. Examples of the ketones include acetone, methyl ethyl ketone, and diethyl ketone. Examples of the ethers include dimethyl ether, ethyl methyl ether, diethyl ether, furan, and tetrahydrofuran. Examples of the nitriles include acrylonitrile. The poor solvent may be at least one selected from the group consisting of methanol, ethanol, and acetone.

In a case where the method of adding a poor solvent to the polymer solution over time is used to bring the polymer solution and the poor solvent into contact with each other, the concentration of the poor solvent in the polymer dispersion liquid to be finally obtained may be 60% by mass or less, 50% by mass or less, 40% by mass or less, 30% by mass or less, or 20% by mass or less from the viewpoint of easily obtaining the polymer in a shorter time. The concentration of the poor solvent in the polymer dispersion liquid may be 10% by mass or more or 20% by mass or more. The content of the poor solvent in the polymer dispersion liquid may be 10% by mass to 60% by mass or 20% by mass to 50% by mass.

In addition, in a case where the method of adding a poor solvent to the polymer solution over time is used to bring the polymer solution and the poor solvent into contact with each other, the addition rate of the poor solvent to the polymer solution may be 20 mass%/min or less or 10 mass%/min or less from the viewpoint that the polymer is easily precipitated in a small particle shape so that impurities are difficult to remain inside the precipitated polymer (that is, the purity of the polymer is increased) and may be 0.1 mass%/min or greater or 1 mass%/min or greater from the viewpoint that the time required for dropwise addition is reduced. The dropping rate of the poor solvent may be 0.1 mass%/min to 20 mass%/min or 1 mass%/min to 10 mass%/min.

The polymer yield in the dispersion step may be 75% by mass or more, 80% by mass or more, 85% by mass or more, or 90% by mass or more.

The shape of the polymer dispersed in the polymer dispersion liquid may be a particle shape or a lump shape.

The transmittance of light having a wavelength of 425 nm through the polymer dispersion liquid at an optical path length of 1 cm is 0.85% or less, and from the viewpoint of sufficiently precipitating a polymer that easily forms a true spherical particle in a dispersion medium in a reversed phase suspension system, the transmittance may be 0.80% or less, 0.70% or less, or less than 0.70%. From the viewpoint of suppressing aggregation of the precipitated polymer to easily form particles having a perfect spherical shape in a dispersion medium in a reversed phase suspension system, the transmittance of light having a wavelength of 425 nm through the polymer dispersion liquid at an optical path length of 1 cm may be 0.01% or greater, 0.10% or greater, 0.20% or greater, or 0.30% or greater. From these viewpoints, the transmittance of light through the polymer dispersion liquid may be 0.01% to 0.80%, 0.01% to 0.70%, or 0.01% or greater and less than 0.70%.

From the viewpoint of suppressing aggregation of the polymer in the dispersion medium and easily forming a spherical particle by easily dispersing the polymer in the dispersion medium in the reversed phase suspension system, the viscosity of the polymer dispersion liquid at 25°C may be 5,000 mPa·s or less, 3,000 mPa·s or less, or 2,000 mPa·s or less. The viscosity of the polymer dispersion liquid at 25°C may be 50 mPa·s or greater, 100 mPa·s or greater, 1,000 mPa·s or greater, 1,500 mPa·s or greater, 3,000 mPa·s or greater, 5,000 mPa·s or greater, or 10,000 mPa·s or greater. From these viewpoints, the viscosity of the polymer dispersion liquid at 25°C may be 50 mPa·s to 5000 mPa·s, 50 mPa·s to 3000 mPa·s, or 50 mPa·s to 2000 mPa·s, 100 mPa·s to 5000 mPa·s, 100 mPa·s to 3000 mPa·s, or 100 mPa·s to 2000 mPa·s.

The weight-average molecular weight of the polymer in the polymer dispersion liquid may be 1,700,000 or greater, 1,800,000 or greater, or 1,900,000 or greater, or may be 2,500,000 or less or 2,400,000 or less. The weight-average molecular weight of the polymer in the polymer dispersion liquid may be 1,700,000 to 2,500,000 or 1,800,000 to 2,400,000.

The median particle diameter of the dried product of the polymer after the addition of the polymer dispersion medium to the dispersion medium in the reversed phase suspension system may be 100 μm or greater, 150 μm or greater, or 200 μm or greater, may be less than 300 μm or 290 μm or less, or may be 100 μm or greater and less than 300 μm.

In the crosslinking step, for example, a solution containing a polymer and a crosslinking agent capable of forming a covalent bond with a functional group contained in the polymer is prepared to crosslink the polymer. In the crosslinking step, the polymer solution may undergo gelation. In this case, the entire polymer solution loses fluidity, and a gel containing a crosslinked polymer and water is formed. In a case where the crosslinking proceeds to the extent that a gel is formed, it is particularly easy to obtain water-absorbent resin particles having high water absorption performance and a small amount of dissolved matter.

Examples of the functional group contained in the polymer include a carboxyl group. In a case where the polymer has a carboxyl group, the polymer is crosslinked by a reaction between the carboxyl group and a crosslinking agent and/or a reaction between the carboxyl groups. The covalent bond may be at least one selected from the group consisting of an ester bond, a thioester bond, an amide bond, an ether bond, and a carbon-carbon bond. The polymer may be crosslinked through at least one group selected from the group consisting of a carboxylic acid ester group, a thioester group, an amide group, an acid anhydride group, an oxyalkylene group, and an oxyarylene group.

Examples of the crosslinking agent include aliphatic polyhydric alcohols such as (poly)ethylene glycol, (poly) propylene glycol, (poly)glycerin, and pentaerythritol; glycidyl compounds such as (poly)ethylene glycol diglycidyl ether, (poly)propylene glycol diglycidyl ether, and (poly)glycerin diglycidyl ether; bisacrylamide compounds such as N,N'-methylene bis(meth)acrylamide; allylated starch; diallyl phthalate; N,N',N''-triallyl isocyanurate; divinylbenzene; ethylenediamine, polyethyleneimine, and glycidyl (meth)acrylate.

From the viewpoint of sufficiently crosslinking the polymer, the amount of the crosslinking agent may be 0.0001 parts by mass or more, 0.001 parts by mass or more, or 0.005 parts by mass or more, and may be 15 parts by mass or less, 10 parts by mass or less, or 5 parts by mass or less per 100 parts by mass of the polymer. The amount of the crosslinking agent may be 0.0001 parts by mass to 15 parts by mass, 0.001 parts by mass to 10 parts by mass, or 0.005 parts by mass to 5 parts by mass per 100 parts by mass of the polymer.

The reaction temperature in a case of crosslinking the polymer is appropriately set depending on the kind, the amount, and the like of the crosslinking agent to be used, but from the viewpoint of sufficiently crosslinking the polymer, the reaction temperature may be 50°C or higher, 80°C or higher, or 100°C or higher, and may be 220°C or 200°C or lower, or 180°C or lower. The reaction temperature in a case of crosslinking the polymer may be 50°C to 220°C, 80°C to 200°C, or 100°C to 180°C.

The reaction time for crosslinking the polymer is appropriately set depending on the kind, the amount, the reaction temperature, and the like of the crosslinking agent to be used, but may be 1 minute to 200 minutes or 5 minutes to 150 minutes from the viewpoint of sufficiently crosslinking the polymer.

After the crosslinked polymer is obtained, a surface crosslinking step of performing surface crosslinking of the crosslinked polymer may be provided. The surface crosslinking can be performed, for example, by adding a crosslinking agent (surface crosslinking agent) for performing the surface crosslinking to the crosslinked polymer and carrying out the reaction.

The surface crosslinking agent may be a compound containing two or more reactive functional groups having reactivity with a functional group (for example, a carboxyl group) contained in the crosslinked polymer. The surface crosslinking agent may be the same as or different from the crosslinking agent in the crosslinking step.

The reactive functional group of the surface crosslinking agent may be a carbonate group, a alcoholic hydroxy group, an epoxy group, a halogeno group in a haloepoxy compound, an isocyanate group, an oxetanyl group, an oxazoline group, or a combination thereof. The carbonate group can react with the other two molecules, and thus is regarded as two reactive functional groups.

Examples of the surface crosslinking agent containing a carbonate group include alkylene carbonate (ethylene carbonate or the like). Examples of surface crosslinking agents containing an alcoholic hydroxyl group include polyol compounds such as ethylene glycol, propylene glycol, 1,4-butanediol, trimethylolpropane, glycerin, polyoxyethylene glycol, polyoxypropylene glycol, and polyglycerin, and hydroxyalkylamide compounds (bis[N,N-di($\beta$-hydroxyethyl)]adi-pamide and the like). Examples of the surface crosslinking agent containing two or more epoxy groups include (poly) ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether. Examples of haloepoxy compounds containing an epoxy group and a halogeno group include epichlorohydrin, epibromohydrin, and $\alpha$-methylepichlorohydrin. Examples of the surface crosslinking agent containing an isocyanate group include 2,4-tolylene diisocyanate and hexamethylene diisocyanate. Examples of surface crosslinking agents containing an oxetanyl group include 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol.

From the viewpoint of sufficiently achieving the surface crosslinking of the crosslinked polymer, the amount of the surface crosslinking agent may be 0.001 parts by mass or more, 0.005 parts by mass or more, or 0.01 parts by mass or more, and may be 5 parts by mass or less, 3 parts by mass or less, or 1 part by mass or less per 100 parts by mass of the crosslinked polymer. The amount of the crosslinking agent may be 0.001 parts by mass to 5 parts by mass, 0.005 parts by mass to 3 parts by mass, or 0.01 parts by mass to 1 part by mass per 100 parts by mass of the polymer.

Water-absorbent resin particles (regenerated water-absorbent resin particles) containing a crosslinked polymer are formed by a method including removing water from the obtained crosslinked polymer. In a case where the reaction solution is an aqueous solution, the water-absorbent resin particles are formed by a method including a drying step of removing water from a lumpy crosslinked polymer formed by gelation of the reaction solution itself and drying the crosslinked polymer to form a dried product and a pulverization step of pulverizing the dried product.

A method of drying the crosslinked polymer may be, for example, a typical method such as a dewatering method such as centrifugation, dehydration using an organic solvent, natural drying, heat drying, air drying, freeze drying, or a combination thereof. From the viewpoint of efficiently removing water, the heating temperature for drying may be 80°C to 220°C, 90°C to 200°C, or 100°C to 180°C.

The moisture content of the dried product may be, for example, 20% by mass or less, 10% by mass or less, or 5% by mass or less. The moisture content of the dried product is based on the total amount of the dried product. That is, the moisture content denotes the proportion of moisture in the dried product. The moisture content of the dried product can be determined as the difference in mass of the crosslinked polymer before and after heating, when the crosslinked polymer containing water is heated at 200°C for 2 hours.

Before drying the crosslinked polymer, a coarsely crushed product having a structure with a small size to some extent may be formed by coarsely crushing the crosslinked polymer. Water can be efficiently removed by forming the coarsely crushed product. The structure constituting the coarsely crushed product can be, for example, an elongated structure, a granular structure (particle), or a combination thereof. The coarsely crushed product may have a plurality of structures having a shape that enables them to pass through a circular hole having a diameter of 10 mm or 7 mm. The elongated structure may be bent, and in a case where the maximum width thereof is 10 mm or less, it can be said that the elongated structure has a shape that enables the structure to pass through a circular hole having a diameter of 10 mm. The granular structure (particle) may be amorphous and may have a shape that enables the structure to pass through a circular hole having a diameter of 10 mm while changing the orientation. Examples of a coarse crusher for coarsely crushing the crosslinked polymer include a kneader (for example, a pressure type kneader or a double-arm type kneader), a meat chopper, a cutter mill, and a pharma mill.

The dried product is pulverized to form water-absorbent resin particles. The pulverization method is not particularly limited. For example, the dried product can be pulverized using a pulverizer such as a centrifugal pulverizer, a roller mill, a stamp mill, a jet mill, a high-speed rotary pulverizer, or a container drive type mill.

The water-absorbent resin particles obtained by pulverization may be classified. The classification denotes an operation of dividing a particle group (powder) into two or more particle groups having different particle size distributions. Some of the water-absorbent resin particles after the classification may be pulverized and classified again.

The classification method is not particularly limited, but may be, for example, screen classification or airflow classification. The screen classification is a method of classifying particles on a screen into particles that pass through meshes of the screen and particles that do not pass through the meshes by vibrating the screen. The screen classification can be performed by using, for example, a vibrating screen, a rotary sifter, a cylindrical stirring screen, a blower sifter, or a Ro-tap shaker. The airflow classification is a method of classifying particles using the flow of air.

The median particle diameter of the powder of the crosslinked polymer obtained by performing pulverization and, as necessary, classification may be, for example, 200 $\mu$m to 500 $\mu$m or 300 $\mu$m to 500 $\mu$m. The particle size distribution may be adjusted by mixing two or more powders having different median particle diameters, which are obtained by the classification.

The centrifuge retention capacity (CRC) of the water-absorbent resin particles may be 15 g/g or greater, 20 g/g or greater, 25 g/g or greater, 30 g/g or greater, 35 g/g or greater, 40 g/g or greater, 45 g/g or greater, or 50 g/g or greater. The water retention ability becomes better as the CRC of the water-absorbent resin particles increases, and the water-absorbing ability is sufficient when the CRC is 15 g/g or greater. The upper limit of the CRC of the water-absorbent resin particles is not particularly limited, but may be, for example, 70 g/g or less, 60 g/g or less, or 55 g/g or less. The CRC of the water-absorbent resin particles may be 15 g/g to 70 g/g, 15 g/g to 60 g/g, or 15 g/g to 55 g/g. The CRC of the water-absorbent resin particles is a value measured by a method described in the examples below.

The amount of the dissolved matter of the water-absorbent resin particles may be 0.2 g/g or less, 0.15 g/g or less, 0.12 or less, 0.1 or less, 0.09 or less, or 0.08 or less. The dissolution of the polymer can be suppressed as the amount of the dissolved matter of the water-absorbent resin particles decreases, and the dissolution of the polymer can be sufficiently suppressed in a case where the amount of the dissolved matter is 0.2 g/g or less. The lower limit of the amount of the dissolved matter of the water-absorbent resin particles is not particularly limited, but may be, for example, 0.01 g/g or greater, 0.02 g/g or greater, or 0.03 g/g or greater. The amount of the dissolved matter of the water-absorbent resin particles may be 0.01 g/g to 0.2 g/g, 0.02 g/g to 0.2 g/g, or 0.03 g/g to 0.2 g/g. The amount of the dissolved matter of the water-absorbent resin particles is a value measured by a method described in the examples below.

The dissolution component index of the water-absorbent resin particles calculated according to the following equation may be $9 \times 10^{-3}$ or less, $8 \times 10^{-3}$ or less, $7 \times 10^{-3}$ or less, $6 \times 10^{-3}$ or less, $5.5 \times 10^{-3}$ or less, $5 \times 10^{-3}$ or less, $4.5 \times 10^{-3}$ or less, $4 \times 10^{-3}$ or less, $3.9 \times 10^{-3}$ or less, $3.8 \times 10^{-3}$ or less, $3.7 \times 10^{-3}$ or less, $3.6 \times 10^{-3}$ or less, or $3.5 \times 10^{-3}$ or less. It can be said that as the dissolution component index of the water-absorbent resin particles decreases, the water-absorbing ability is sufficient, and the dissolution of the polymer can be suppressed. The dissolution component index of the water-absorbent resin particles may be $1 \times 10^{-3}$ or greater, $1.5 \times 10^{-3}$ or greater, $2 \times 10^{-3}$ or greater, $2.5 \times 10^{-3}$ or greater, $3 \times 10^{-3}$ or greater, $3.1 \times 10^{-3}$ or greater, $3.2 \times 10^{-3}$ or greater, $3.3 \times 10^{-3}$ or greater, $3.4 \times 10^{-3}$ or greater, or $3.5 \times 10^{-3}$ or greater. The dissolution component index of the water-absorbent resin particles may be $1 \times 10^{-3}$ to $9 \times 10^{-3}$, $2 \times 10^{-3}$ to $6 \times 10^{-3}$, or $3 \times 10^{-3}$ to $5 \times 10^{-3}$. The dissolution component index is represented by the following expression.

Dissolution component index = dissolved matter [g/g]/CRC [g/g]

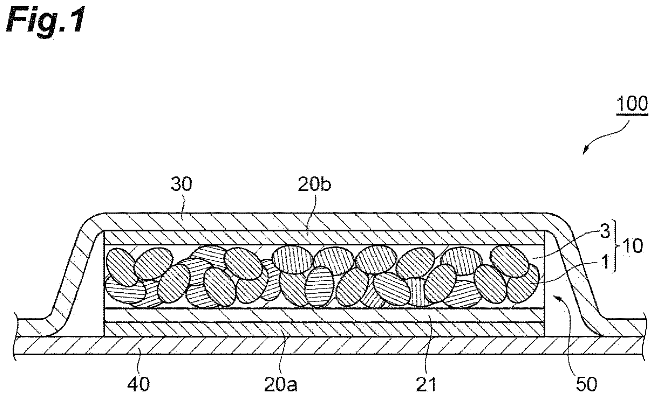

Fig. 1 is a cross-sectional view showing an example of an absorbent article having an absorbent body containing water-absorbent resin particles. An absorbent article 100 shown in Fig. 1 includes an water-absorbent sheet 50 having a film-like absorbent body 10, a liquid permeable sheet 30, and a liquid impermeable sheet 40.

The water-absorbent sheet 50 has an absorbent body 10 containing a powder of water-absorbent resin particles 1 and two sheets of core wrap sheets 20a and 20b. The absorbent body 10 is disposed inside the core wrap sheets 20a and 20b. The shape of the absorbent body 10 is retained by being sandwiched between the two core wrap sheets 20a and 20b. The core wrap sheets 20a and 20b may be two sheets, one turned-back sheet, or one bag body. A sheet member in which other constituent members are not provided on the outside of the core wrap sheets 20a and 20b that wrap the absorbent body 10 is also particularly referred to as a water-absorbent sheet.

The absorbent body 10 is a constituent member which mainly contains a powder of the water-absorbent resin particles 1 and in which the shape is retained to be constant. The absorbent body 10 may or may not include a fibrous substance 3 in addition to the powder of the water-absorbent resin particles 1. The content of the water-absorbent resin particles 1 in the absorbent body 10 may be 50% by mass or more and 100% by mass or less, 60% by mass or more and 100% by mass or less, 70% by mass or more and 100% by mass or less, 80% by mass or more and 100% by mass or less, or 90% by mass or more and 100% by mass or less with respect to the mass of the absorbent body 10.

The thickness of the absorbent body 10 may be, for example, 20 mm or less, 15 mm or less, 10 mm or less, 5 mm or less, 4 mm or less, or 3 mm or less, or may be 0.1 mm or more or 0.3 mm or more. The thickness of the absorbent body 10 may be 0.1 mm or more and 20 mm or less. The mass of the absorbent body 10 per unit area may be 1000 $g/m^2$ or less, 800 $g/m^2$ or less, or 600 $g/m^2$ or less, or may be 100 $g/m^2$ or greater.

The fibrous substance 3 can be, for example, cellulosic fibers, synthetic fibers, or a combination thereof. Examples of the cellulosic fibers include pulverized wood pulp, cotton, cotton linter, rayon, and cellulose acetate. Examples of the synthetic fibers include polyamide fibers, polyester fibers, and polyolefin fibers. The fibrous substance may be hydrophilic fiber (for example, pulp).

The absorbent body 10 may further contain an inorganic powder (for example, amorphous silica), a deodorant, an antibacterial agent, a fragrance, or the like. In a case where the water-absorbent resin particles 1 include inorganic particles, the absorbent body 10 may contain an inorganic powder in addition to the inorganic particles of the water-absorbent resin particles 1.

The water-absorbent sheet 50 may further contain an adhesive 21 sandwiched between the core wrap sheet 20a and the absorbent body 10. An adhesive layer may be sandwiched between the core wrap sheets 20a and 20b on both sides and the absorbent body 10. The adhesive 21 is not particularly limited, and may be, for example, a hot melt adhesive.

The core wrap sheets 20a and 20b may be, for example, non-woven fabrics. The two core wrap sheets 20a and 20b can be non-woven fabrics that are the same as or different from each other. The non-woven fabrics may be non-woven fabrics (short-fiber non-woven fabrics) formed of short fibers (that is, staple fibers), or may be non-woven fabrics (long-fiber non-woven fabrics) formed of long fibers (that is, filaments). The staples are not limited thereto, but may have a typical fiber length of several hundred mm or less.

The core wrap sheets 20a and 20b may be thermal bonded non-woven fabrics, air-through non-woven fabrics, resin bonded non-woven fabrics, spunbond non-woven fabrics, melt-blown non-woven fabrics, air-laid non-woven fabrics, spunlace non-woven fabrics, point-bonded non-woven fabrics, or a laminate containing two or more non-woven fabrics selected from these non-woven fabrics.

The non-woven fabric used as the core wrap sheets 20a and 20b can be non-woven fabrics formed of synthetic fibers, natural fibers, or a combination thereof. Examples of the synthetic fibers include fibers containing synthetic resins selected from polyolefin such as polyethylene (PE) and polypropylene (PP), polyester such as polyethylene terephthalate (PET), polytrimethylene terephthalate (PTT), and polyethylene naphthalate (PEN), polyamide such as nylon, and rayon. Examples of the natural fibers include fibers containing cotton, silk, hemp, or pulp (cellulose). Fibers forming the non-woven fabrics may be polyolefin fibers, polyester fibers, or a combination thereof. The core wrap sheets 20a and 20b may be tissue paper.

The water-absorbent sheet 50 may be used for producing various other absorbent articles. Examples of the absorbent articles include diapers (for example, paper diapers), toilet training pants, incontinence pads, sanitary materials (sanitary napkins, tampons, and the like), sweat pads, pet sheets, portable toilet members, and animal excrement treatment materials. The absorbent body constituting these absorbent articles frequently moves or is deformed due to the movement or the like of a user of the absorbent article.

The liquid permeable sheet 30 is disposed at the position of the outermost layer on a side where the liquid to be absorbed enters. The liquid permeable sheet 30 is disposed outside the core wrap sheet 20b in a state of being in contact with the core wrap sheet 20b. The liquid impermeable sheet 40 is disposed at the position of the outermost layer on the opposite side to the liquid permeable sheet 30 in the absorbent article 100. The liquid impermeable sheet 40 is disposed outside the core wrap sheet 20a in a state of being in contact with the core wrap sheet 20a. The liquid permeable sheet 30 and the liquid impermeable sheet 40 have a main surface wider than the main surface of the water-absorbent sheet 50, and outer edges of the liquid permeable sheet 30 and the liquid impermeable sheet 40 extend around the absorbent body 10 and the core wrap sheets 20a and 20b. However, the magnitude relationship between the absorbent body 10, the core wrap sheets 20a and 20b, the liquid permeable sheet 30, and the liquid impermeable sheet 40 is not particularly limited, and is appropriately adjusted depending on the applications of the absorbent article or the like.

The liquid permeable sheet 30 may be a non-woven fabric. The non-woven fabric used as the liquid permeable sheet 30 may have appropriate hydrophilicity from the viewpoint of the liquid absorption performance of the absorbent article. From this viewpoint, the liquid permeable sheet 30 may be a non-woven fabric having a hydrophilicity of 5 to 200, which is measured by a measuring method of the Japan Technical Association of Pulp and Paper Industry Test Method No. 68 (2000) of the Japan Technical Association of Pulp and Paper Industry. The hydrophilicity of the non-woven fabric may be 10 to 150. The details of Pulp and Paper Test Method No. 68 can be referred to, for example, WO2011/086843.

The non-woven fabric having hydrophilicity may be formed of fibers, such as rayon fibers exhibiting an appropriate hydrophilicity, or may be formed of fibers obtained by performing a hydrophilic treatment on hydrophobic chemical fibers such as polyolefin fibers and polyester fibers. Examples of a method of obtaining a non-woven fabric including hydrophobic chemical fibers that have been subjected to a hydrophilic treatment include a method of obtaining a non-woven fabric by a spunbond method using a mixture obtained by mixing a hydrophilic agent with hydrophobic chemical fibers, a method of accompanying a hydrophilic agent in preparing a spunbond non-woven fabric with hydrophobic chemical fibers, and a method of impregnating a spunbond non-woven fabric obtained using hydrophobic chemical fibers with a hydrophilic agent. As the hydrophilic agent, an anionic surfactant such as an aliphatic sulfonate or a higher alcohol sulfate ester salt, a cationic surfactant such as a quaternary ammonium salt, a nonionic surfactant such as polyethylene glycol fatty acid ester, polyglycerin fatty acid ester, or sorbitan fatty acid ester, a silicone surfactant such as polyoxyalkylene-modified silicone, a stain release agent formed of a polyester-based, polyamide-based, acrylic-based, or urethane-based resin, or the like is used.

From the viewpoint of imparting satisfactory liquid permeability, flexibility, strength, and cushioning properties to the absorbent article and from the viewpoint of accelerating the liquid permeation rate of the absorbent article, the basis weight (the mass per unit area) of the non-woven fabric used as the liquid permeable sheet 30 may be 5 g/m$^2$ to 200 g/m$^2$, 8 g/m$^2$ to 150 g/m$^2$, or 10 g/m$^2$ to 100 g/m$^2$. The thickness of the liquid permeable sheet 30 may be 20 μm to 1400 μm, 50 μm to 1200 μm, or 80 μm to 1000 μm.

The liquid impermeable sheet 40 prevents a liquid absorbed by the absorbent body 10 from leaking to the outside from the liquid impermeable sheet 40 side. The liquid impermeable sheet 40 may be a resin sheet or a non-woven fabric. The resin sheet may be a sheet formed of a synthetic resin such as polyethylene, polypropylene, and polyvinyl chloride. The non-woven fabric may be a spunbond/melt-blown/spunbond (SMS) non-woven fabric in which a waterproof melt-blown non-woven fabric is sandwiched between high-strength spunbond non-woven fabrics. The liquid impermeable sheet 40 may be a composite sheet of a resin sheet and a non-woven fabric (for example, spunbond non-woven fabric, spunlace non-woven fabric). The liquid impermeable sheet 40 may have breathability from the viewpoint that dampness generated at the time of wearing is reduced, and thereby discomfort to a wearer can be reduced. As the liquid impermeable sheet 40 having breathability, it is possible to use, for example, a sheet of low-density polyethylene (LDPE) resin.

From the viewpoint of ensuring the flexibility so that the feeling of wearing the absorbent article is not impaired, the basis weight (mass per unit area) of the liquid impermeable sheet 40 may be 10 g/m$^2$ to 50 g/m$^2$.

**Examples**

Hereinafter, the present invention will be described in more detail with reference to the Examples.

(Example 1)

A commercially available paper diaper (manufactured by Kao Corporation, Merries Pants Smooth Air Through, L size) was prepared, and water-absorbent resin particles containing a crosslinked polymer containing acrylic acid and an acrylic acid salt as monomer units were collected from the paper diaper.

A reflux cooling device, a dropping funnel, a nitrogen gas introduction pipe, and a stirrer (a 4 L round-bottomed cylindrical separable flask equipped with a stirring blade having four inclined paddle blades with a blade diameter of 50 mm in two stages, manufactured by DURAN) were prepared. 45 g of the water-absorbent resin particles collected from a paper diaper were weighed in a separable flask, 855 g of ion-exchanged water was added thereto, and the mixture was allowed to stand for 5 minutes to swell the water-absorbent resin particles. Next, 1200 g of a 1.5 mol/kg sodium hydroxide aqueous solution was added to a separable flask, and the crosslinked structure of the crosslinked polymer of the water-absorbent resin particles was cleaved while the internal temperature was maintained at 80°C for 24 hours and the solution was stirred at 160 rpm with a stirring blade. A decomposition solution (aqueous solution of water-soluble recycled polymer, pH 13) containing a polymer which was a water-absorbent resin solubilized by cleavage of the crosslinked structure was obtained. Further, in a case where the weight-average molecular weight of the water-soluble recycled polymer was measured by the above-described method, the Mw thereof was 1,950,000.

The obtained decomposition solution was filtered through a filter (UHP-150K, manufactured by ADVANTEC) and an ultrafiltration membrane (manufactured by Nitto Denko Corporation, molecular weight cut-off: 500,000). Next, the residue on the filtration membrane was dried, thereby obtaining 39 g of a water-soluble recycled polymer from the decomposition solution. Further, in a case of performing filtration, in order to reduce damage to the ultrafiltration membrane, the pH of the decomposition solution was adjusted to 7 using sulfuric acid (manufactured by Hayashi Pure Chemical Ind., Ltd., special grade) before filtration.

285 g of ion-exchanged water was weighed in a 500 mL polybeaker, and the ion-exchanged water in the polybeaker was stirred at 400 rpm by a pair of paddles (blade diameter: 75 mm) attached to a jar tester (MJS-10-700, manufactured by Daido Kogyo Co., Ltd.). Next, 15 g of a water-soluble recycled polymer was added to the ion-exchanged water, and the solution was stirred for 60 minutes, thereby preparing a polymer solution which was an aqueous solution of 5% by mass of the water-soluble recycled polymer.

The prepared polymer solution was stirred at 650 rpm for 20 minutes with a high-speed stirrer (MK-H4, manufactured by Panasonic Corporation), and stirring was continued for 5 minutes while 0.75 g (0.043 mmol) of an aqueous solution containing 1% by mass of ethylene glycol diglycidyl ether was added thereto as a crosslinking agent.

The polymer solution after being stirred with a high-speed stirrer was transferred to a bat (20 cm × 16 cm) coated with Teflon (registered trademark) and heated at 115°C for 60 minutes with a blast dryer (FV-320, manufactured by ADVANTEC) to crosslink the water-soluble recycled polymer with the crosslinking agent. As the crosslinking of the water-soluble recycled polymer progressed, the entire polymer solution gelled. A lumpy gel containing the crosslinked polymer and water was taken out from the bat, and the gel was roughly cut into a size of 3 cm square in order to efficiently dry the gel. The cut gel was transferred to a bat again and dried by being heated at 115°C for 120 minutes with a blast dryer to obtain a water-soluble recycled polymer crosslinked substance.

The obtained water-soluble recycled polymer crosslinked substance was subjected to a pulverization treatment at 6000 rpm with a 1 mm screen using a centrifugal pulverizer (ZM-2000, manufactured by Retsch), thereby obtaining 11.5 g of regenerated water-absorbent resin particles having a median particle diameter of 384 $\mu$m.

(Example 2)

11.0 g of regenerated water-absorbent resin particles having a median particle diameter of 388 $\mu$m were obtained by performing the same operation as in Example 1 except that 1.2 g (0.069 mmol) of a 1 mass% ethylene glycol diglycidyl ether aqueous solution was added as a crosslinking agent.

(Example 3)

10.8 g of regenerated water-absorbent resin particles having a median particle diameter of 396 $\mu$m were obtained by performing the same operation as in Example 1 except that 7.6 g (2.19 mmol) of a 5 wt% ethylene glycol diglycidyl ether aqueous solution was added as a crosslinking agent.

(Example 4)

A 1 L polybeaker equipped with a liquid feeding pump and a stirring blade (having a surface coated with a fluororesin), the stirring blade having four inclined paddle blades with a blade diameter of 70 mm arranged in two stages, was prepared. 50.00 g of a 6.0 mass% sodium polyacrylate (degree of neutralization: 70 mol%) aqueous solution was added to the polybeaker as a polymer solution, and the rotation speed of the stirrer was set to 1000 r/min. To the polymer solution in this polybeaker, 50.00 g of a 70.0 mass% ethanol aqueous solution was added dropwise using a liquid feeding pump at 7.9 mass%/min (10 ml/min), thereby obtaining a turbid colloidal solution (polymer dispersion liquid). 1.50 g (0.0086 mmol) of a 0.1 mass% ethylene glycol diglycidyl ether aqueous solution was added to the polybeaker as a crosslinking agent.

A round-bottomed cylindrical separable flask having an inner diameter of 100 mm, equipped with a reflux cooling device, a dropping funnel, a nitrogen gas introduction pipe, and a stirring blade assembly (having a surface coated with a fluororesin) having four inclined paddle blades with a blade diameter of 50 mm in three stages as a stirrer, was prepared. 0.50 g of a sucrose stearic acid ester (RYOTO Sugar Ester S-370, manufactured by Mitsubishi Chemical Foods Corporation, HLB: 3) as a surfactant and 0.50 g of a maleic anhydride-modified ethylene-propylene copolymer (Hi-WAX 1105A, manufactured by Mitsui Chemicals, Inc.) as a polymer-based dispersant were added to the flask, and the rotation speed of the stirrer was set to 500 r/min. 500.00 g of n-heptane was added to the separable flask, the temperature was increased to 85°C to dissolve the surfactant and the dispersant, and the internal temperature was set to 75°C. The rotation speed of the stirrer was set to 1,000 r/min, the colloidal solution after the addition of the crosslinking agent was added to the separable flask, and a crosslinking reaction was carried out for 60 minutes. Thereafter, the flask was immersed in an oil bath set to 125°C, and n-heptane and water were evaporated to dried, and further the resulting product was passed through a standard sieve with an opening of 300 $\mu$m, thereby obtaining 1.14 g of water-absorbent resin particles having a median particle diameter of 197 $\mu$m.

(Example 5)

1.88 g of regenerated water-absorbent resin particles having a median particle diameter of 176 $\mu$m were obtained by performing the same operation as in Example 4 except that 4.3 g (0.024 mmol) of a 0.1 mass% ethylene glycol diglycidyl ether aqueous solution was added as a crosslinking agent.

(Example 6)

1.40 g of regenerated water-absorbent resin particles having a median particle diameter of 150 $\mu$m were obtained by performing the same operation as in Example 4 except that 0.55 g (0.063 mmol) of a 2.0 mass% ethylene glycol diglycidyl ether aqueous solution was added as a crosslinking agent.

(Comparative Example 1)

The water-soluble recycled polymer was crosslinked through an ionic bond using calcium ions by performing the same operation as in Example 1 except that the aqueous solution containing 1% by mass of ethylene glycol diglycidyl ether was changed to 7.2 g (2.45 mmol) of a 5 mass% aqueous solution containing calcium chloride dihydrate (manufactured by Kanto Chemical Co., Inc., special grade) as a crosslinking agent. As the crosslinking progressed, the viscosity of the polymer solution increased, but the fluidity was maintained and gelation did not occur. The generation of aggregates was not observed in the polymer solution. The polymer solution having a high viscosity was dried by being heated at 115°C for 120 minutes with a forced-air dryer in a bat, thereby obtaining a water-soluble recycled polymer crosslinked substance. The obtained water-soluble recycled polymer crosslinked substance was subjected to a pulverization treatment at 6000 rpm with a 1 mm screen using a centrifugal pulverizer (ZM-2000, manufactured by Retsch), thereby obtaining 10.5 g of regenerated water-absorbent resin particles having a median particle diameter of 398 $\mu$m.

(Comparative Example 2)

10.6 g of regenerated water-absorbent resin particles having a median particle diameter of 378 $\mu$m were obtained by performing the same operation as in Comparative Example 1 except that 30 g (10.2 mmol) of a 5 mass% aqueous solution of a calcium chloride dihydrate (manufactured by Kanto Chemical Co., Inc., special grade) was used as a crosslinking agent. As the crosslinking progressed, the generation of aggregates was observed in the polymer solution.

(Comparative Example 3)

11.1 g of regenerated water-absorbent resin particles having a median particle diameter of 377 $\mu$m were obtained by performing the same operation as in Example 1 except that 15 g (2.19 mmol) of a 5 mass% aqueous solution of aluminium sulfate (manufactured by Kanto Chemical Co., Inc., Cica Special Grade) was used as a crosslinking agent.

(Comparative Example 4)

11.2 g of regenerated water-absorbent resin particles having a median particle diameter of 399 $\mu$m were obtained by performing the same operation as in Comparative Example 1 except that 30 g (4.38 mmol) of a 5 mass% aqueous solution of aluminum sulfate (manufactured by Kanto Chemical Co., Inc., Cica Special Grade) was used as a crosslinking agent. As the crosslinking progressed, the generation of aggregates was observed in the polymer solution.

(Reference Example 1)

Water-absorbent resin particles were collected from a paper diaper (manufactured by Kao Corporation, Merries Pants Smooth Air Through, L size) used in the examples.

<Evaluation method>

The centrifuge retention capacity (CRC), the dissolved matter, the dissolution component index, and the median particle diameter (particle size distribution) of the obtained water-absorbent resin particles were measured according to the following evaluation methods. The measurement results of the centrifuge retention capacity (CRC), the dissolved matter, and the dissolution component index of the water-absorbent resin particles are listed in Table 1. Further, the amount (mmol) of the bond in Table 1 is calculated as the product of the amount (mmol) of the crosslinking agent and the number of functional groups serving as crosslinking points of the crosslinking agent.

[Centrifuge retention capacity (CRC)]

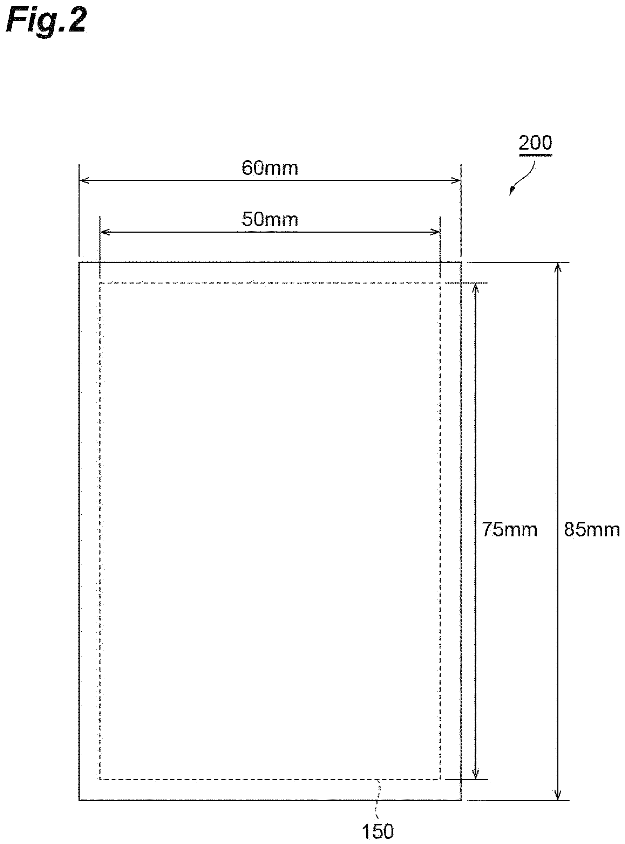

The centrifuge retention capacity (CRC) was measured by the following procedures with reference to the EDANA method (NWSP 241.0.R2 (19)). The measurement was performed under an environment of a temperature of 25 $\pm$ 2°C and a relative humidity of 50 $\pm$ 10%.
A non-woven fabric bag having a size of 60 mm $\times$ 85 mm was prepared by compression-bonding two sheets of non-woven fabric having a size of 60 mm $\times$ 85 mm to each other by heat sealing at end portions having a width of 5 mm along each of three sides. About 0.2 g of the precisely weighed recycled water-absorbent resin particles were stored in the non-woven fabric bag. Next, the non-woven fabric bag shown in Fig. 2 was closed by sealing the non-woven fabrics by heat sealing at the end portion of one side of the opening.
A plurality of non-woven fabric bags containing water-absorbent resin particles were floated on 500 g of physiological saline stored in a stainless steel bat (240 mm × 320 mm × 45 mm) such that the non-woven fabric bags did not overlap each other, thereby completely wetting the entire non-woven fabric bag. The entire non-woven fabric bag was immersed in the physiological saline using a spatula 1 minute after the non-woven fabric bag was floated on the physiological saline.

After 30 minutes from the start of placing the non-woven fabric bag on the physiological saline, that is, after a total time of 1 minute of placing the non-woven fabric bag and 29 minutes of the immersion, the non-woven fabric bag was taken out from the physiological saline. The non-woven fabric bag that had been taken out was dehydrated with a centrifuge (manufactured by KOKUSAN Co., Ltd., model number: H-122) at a centrifugal force of 250 G for 3 minutes. After the dehydration, the mass Wc [g] of the non-woven fabric bag including the mass of the gel was weighed. The above-described operation was performed on a non-woven fabric bag that did not contain the regenerated water-absorbent resin particles to be measured, and the mass B [g] of the non-woven fabric bag after the dehydration was measured. CRC [g/g] was calculated according to the following equation. Sc [g] represents a precisely weighed value of 0.2 g of the mass of the regenerated water-absorbent resin particles to be measured.

$$CRC \ [g/g] = \{(Wc - B) - Sc\}/Sc$$

[Dissolved matter and dissolution component index]

The amount of the dissolved matter of the regenerated water-absorbent resin was measured in an environment of a temperature of 25°C ± 2°C and a humidity of 50% ± 10%. 500 g of physiological saline in a 500 mL beaker was stirred with a stirrer (cylindrical shape having a diameter of 8 mm and a length of 30 mm, no ring) rotating at 600 rpm. The temperature of the physiological saline was 25°C. 2.000 g of the regenerated water-absorbent resin particles were added thereto, and the dispersion liquid containing the regenerated water-absorbent resin particles was stirred for 3 hours. The dispersion liquid was filtered through a standard screen having an opening of 75 μm, and the filtrate was collected. 80 g of the obtained filtrate was weighed in a 100 mL beaker which had been dried to constant weight in advance at 140°C. The filtrate in the beaker was heated in a blast dryer (FV-320, manufactured by ADVANTEC) at 140°C for 15 hours to remove the moisture, and the mass Wa (g) of the remaining solid component was measured. A blank test was performed by the same operation as the operation described above without putting the regenerated water-absorbent resin particles into physiological saline, and the mass Wb (g) of the solid component remaining in the beaker was measured. The amount of dissolved matter was calculated according to the following equation. Further, the dissolution component index was calculated according to the following equation from the above-described CRC and the amount of dissolved matter.

$$Dissolved \ matter \ [g/g] = ((Wa - Wb)/80) \times 500/2$$

Dissolution component index = dissolved matter [g/g]/CRC [g/g]

[Medium particle size (particle size distribution)]

The median particle diameter of the regenerated water-absorbent resin particles was measured in a normal-temperature and normal-humidity environment according to the following procedures.

Using a continuous fully automatic acoustic oscillation type sieving measuring machine (ROBOT SHIFTER RPS-205, manufactured by SeishinEnterprise Co., Ltd.), the particle size distribution of 2 g of the regenerated water-absorbent resin particles was measured using JIS standard sieves with an opening of 850 μm, an opening of 600 μm, an opening of 500 μm, an opening of 425 μm, an opening of 300 μm, an opening of 250 μm, and an opening of 180 μm, and a receiver. The regenerated water-absorbent resin particles were fed to the topmost sieve of the combined standard sieves and shaken for 5 minutes to carry out classification. By integrating the particle mass on the standard sieves in order from the side with the largest particle diameter, a relationship between the opening of the standard sieve and the integrated value of the mass percentage of the particles remaining on the standard sieves was plotted on a logarithmic probability paper. The plots on the probability paper were connected in a straight line to obtain the particle diameter corresponding to a cumulative mass percentage of 50% by mass as the median particle diameter.

[Table 1]

| | Crosslinking agent | Amount of crosslinking agent (mmol) | Amount of bond (mmol) | Appearance of polymer solution after addition of crosslinking agent | CRC (g/g) | Dissolved matter (g/g) | dissolution component index |
|---|---|---|---|---|---|---|---|
| Example 1 | Ethylene glycol diglycidyl ether | 0.043 | 0.086 | Gelation | 51.6 | 0.197 | $3.8 \times 10^{-3}$ |
| Example 2 | Ethylene glycol diglycidyl ether | 0.069 | 0.138 | Gelation | 40.6 | 0.141 | $3.5 \times 10^{-3}$ |
| Example 3 | Ethylene glycol diglycidyl ether | 2.19 | 4.38 | Gelation | 17.1 | 0.077 | $4.5 \times 10^{-3}$ |
| Example 4 | Ethylene glycol diglycidyl ether | 0.0086 | 0.017 | Gelation in n-heptane | 56.5 | 0.192 | $3.4 \times 10^{-3}$ |
| Example 5 | Ethylene glycol diglycidyl ether | 0.024 | 0.049 | Gelation in n-heptane | 30.0 | 0.137 | $4.6 \times 10^{-3}$ |
| Example 6 | Ethylene glycol diglycidyl ether | 0.063 | 0.126 | Gelation in n-heptane | 21.9 | 0.112 | $5.1 \times 10^{-3}$ |
| Comparative Example 1 | Calcium chloride | 2.45 | 4.90 | Aggregates were not found | 1.1 | 0.868 | $7.9 \times 10^{-1}$ |
| Comparative Example 2 | Calcium chloride | 10.2 | 20.2 | Small amount of aggregates were found | 9.7 | 0.874 | $9.0 \times 10^{-2}$ |
| Comparative Example 3 | Aluminum sulfate | 2.19 | 6.57 | Scattered aggregates were found | 22.2 | 0.217 | $9.8 \times 10^{-3}$ |
| Comparative Example 4 | Aluminum sulfate | 4.38 | 13.1 | Scattered aggregates were found | 12.6 | 0.152 | $1.2 \times 10^{-2}$ |
| Reference Example 1 | Water-absorbent resin particles collected from paper diaper used in examples | | | | 34.1 | 0.233 | $6.8 \times 10^{-3}$ |

It was found that the absorbent article formed of the regenerated water-absorbent resin particles obtained in the examples has high water absorption performance and can suppress dissolution of the polymer.

**Reference Signs List**

1: water-absorbent resin particle
10: absorbent body
20a, 20b: core wrap sheet
30: liquid permeable sheet
40: liquid impermeable sheet
50: water-absorbent sheet

100: absorbent article
150: heat seal portion
200: non-woven fabric bag

**Claims**

1.  A production method for water-absorbent resin particles comprising a crosslinked polymer, the production method comprising:

    a preparation step of preparing a polymer solution that comprises a polymer and a solvent; and
    a crosslinking step of crosslinking the polymer through a covalent bond to form the crosslinked polymer.

2.  The production method according to Claim 1,
    wherein the preparation step comprises cleaving a crosslinked structure of a crosslinked polymer in a water-absorbent resin for recycling that comprises the crosslinked polymer, to obtain the polymer.

3.  The production method according to Claim 2,
    wherein the preparation step further includes collecting the water-absorbent resin for recycling from an absorbent article.

4.  The production method according to any one of Claims 1 to 3,
    wherein the polymer has a weight-average molecular weight of 100,000 or more.

5.  The production method according to any one of Claims 1 to 3,
    wherein in the crosslinking step, the polymer solution is gelled.

6.  The production method according to any one of Claims 1 to 3,

    wherein the polymer comprises a carboxyl group, and
    the polymer is crosslinked by a reaction between the carboxyl group and a crosslinking agent and/or a reaction between the carboxyl groups.

7.  The production method according to any one of Claims 1 to 3,
    wherein the polymer is crosslinked through at least one group selected from the group consisting of a carboxylic acid ester group, a thioester group, an amide group, an acid anhydride group, an oxyalkylene group, and an oxyarylene group.

8.  Water-absorbent resin particles,
    wherein a dissolution component index represented by the following equation is $2 \times 10^{-3}$ to $6 \times 10^{-3}$,

    dissolution component index = dissolved matter (g/g)/CRC (g/g).

9.  An absorbent body comprising:
    the water-absorbent resin particles according to Claim 8.

10. An absorbent article comprising:
    the absorbent body according to Claim 9.

## Fig.1

# *Fig.2*

# EP 4 667 517 A1

## INTERNATIONAL SEARCH REPORT

| | International application No. |
|---|---|
| | **PCT/JP2024/012891** |

### A. CLASSIFICATION OF SUBJECT MATTER

*C08J 11/16*(2006.01)i; *A61F 13/15*(2006.01)i; *A61F 13/53*(2006.01)i; *A61L 15/24*(2006.01)i; *A61L 15/60*(2006.01)i
FI:   C08J11/16 ZAB; A61F13/15 320; A61F13/53 300; A61L15/24 100; A61L15/60 100

According to International Patent Classification (IPC) or to both national classification and IPC

### B. FIELDS SEARCHED

Minimum documentation searched (classification system followed by classification symbols)

C08J11/16; A61F13/15; A61F13/53; A61L15/24; A61L15/60

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2020-49398 A (LIVEDO CORPORATION) 02 April 2020 (2020-04-02) paragraphs [0009]-[0091] | 1-7 |
| X | JP 2012-219172 A (MITSUI CHEMICALS, INC.) 12 November 2012 (2012-11-12) paragraphs [0024]-[0108], [0135]-[0157] | 1-7 |
| A | WO 2021/162082 A1 (NIPPON SHOKUBAI CO., LTD.) 19 August 2021 (2021-08-19) | 1-7 |

☐ Further documents are listed in the continuation of Box C.          ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **18 June 2024** | **25 June 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT

| International application No. |
|---|
| **PCT/JP2024/012891** |

| **Box No. III** **Observations where unity of invention is lacking (Continuation of item 3 of first sheet)** |
|---|

This International Searching Authority found multiple inventions in this international application, as follows:

Document 1: JP 2020-49398 A (LIVEDO CORPORATION) 02 April 2020 (2020-04-02), paragraphs [0009]-[0091] (Family: none)

(Invention 1) Claims 1-7
    Document 1 (paragraphs [0029]-[0039]) discloses "a method for producing water absorbent resin particles containing a crosslinked polymer, the method comprising a preparation step for preparing a polymer solution containing a polymer and a solvent, and a crosslinking step for forming the crosslinked polymer by crosslinking the polymer through a covalent bond."
Claim 1 lacks novelty in light of document 1, and thus do not have a special technical feature. In addition, claims 2-4 are simple design modification of the document 1 (paragraphs [0029]-[0088]), do not make a contribution over the prior art, and thus cannot be a special technical feature. Thus, claims 1-4 are classified as invention 1.
    Also, claims 5-7 are dependent on claim 1 and inventively related to claim 1, and are thus classified as invention 1.
(Invention 2) Claims 8-10
    Claims 8-10 share the common technical feature of a water absorbent resin particles with claim 1 classified as invention 1. However, said technical feature does not make a contribution over the prior art in light of the disclosures of document 1 (paragraphs [0009]-[0091]) and thus cannot be said to be a special technical feature. Also, there are no other the same as or corresponding special technical features between these inventions.
    In addition, claims 8-10 are not dependent on claim 1. Furthermore, claims 8-10 are not substantially identical to or similarly closely related to any of the claims classified as invention 1.
    Therefore, claims 8-10 cannot be classified as invention 1.
    In addition, claims 8-10 have the special technical feature of a "water absorbent resin particles having a dissolution component index of $2\times10^{-3}$ to $6\times10^{-3}$ expressed by the formula below:
dissolved component index = dissolved component (g/g)/CRC(g/g)"and are thus classified as invention 2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-7**

**Remark on Protest**     ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
Information on patent family members

International application No.

**PCT/JP2024/012891**

| Patent document cited in search report | | | Publication date (day/month/year) | Patent family member(s) | | | Publication date (day/month/year) |
|---|---|---|---|---|---|---|---|
| JP | 2020-49398 | A | 02 April 2020 | (Family: none) | | | |
| JP | 2012-219172 | A | 12 November 2012 | (Family: none) | | | |
| WO | 2021/162082 | A1 | 19 August 2021 | US | 2023/0147797 | A1 | |
| | | | | EP | 4104942 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020049398 A

- WO 2011086843 A